# EUROPÄISCHE PATENTSCHRIFT

(11) **EP 1 294 385 B1**
(45) Veröffentlichungstag und Bekanntmachung des Hinweises auf die Patenterteilung: **04.04.2007**
(21) Anmeldenummer: 01957822.8
(22) Anmeldetag: 02.06.2001
(51) Int. Cl.: A61K 35/14, A61K 38/42, A61P 43/00

(54) **VERFAHREN ZUR HERSTELLUNG KÜNSTLICHER SAUERSTOFFTRÄGER AUS KOVALENT VERNETZTEN HÄMOGLOBINEN MIT VERBESSERTEN FUNKTIONELLEN EIGENSCHAFTEN DURCH VERNETZUNG IN ANWESENHEIT CHEMISCH NICHT REAGIERENDER EFFEKTOREN DER SAUERSTOFFAFFINITÄT DER HÄMOGLOBINE**
METHOD FOR THE PRODUCTION OF ARTIFICIAL OXYGEN CARRIERS FROM COVALENTLY CROSS LINKING HAEMOGLOBIN WITH IMPROVED FUNCTIONAL PROPERTIES OF HAEMOGLOBIN BY CROSS-LINKING IN THE PRESENCE OF CHEMICALLY NON-REACTING EFFECTORS OF THE OXYGEN AFFINITY OF THE HAEMOGLOBIN
PROCEDE DE FABRICATION DE PORTEURS D'OXYGENE ARTIFICIELS A PARTIR D'HEMOGLOBINES RETICULEES DE MANIERE COVALENTE AYANT DES PROPRIETES FONCTIONNELLES AMELIOREES PAR RETICULATION EN PRESENCE D'EFFECTEURS DE L'AFFINITE A L'OXYGENE DE L'HEMOGLOBINE, NE REAGISSANT PAS CHIMIQUEMENT

(30) Priorität: 29.06.2000 DE 10031742
(43) Veröffentlichungstag der Anmeldung: 26.03.2003
(73) Patentinhaber: SanguiBioTech GmbH, 58455 Witten an der Ruhr (DE)
(72) Erfinder: BARNIKOL, Wolfgang, 55128 Mainz (DE)
(74) Vertreter: Müller, Claudia
(86) Internationale Anmeldenummer: PCT/EP2001/006329
(87) Internationale Veröffentlichungsnummer: WO 2002/000229

(56) Entgegenhaltungen:
- US-A- 4 600 531
- US-A- 5 250 665
- US-A- 5 387 672
- US-A- 5 532 352
- KLETT D., ET AL.: "Fixation of aldehydic dextrans onto human deoxyhemoglobin" BIOPOLYMERS, Bd. 32, Nr. 5, 1992, Seiten 517-522, XP001029802

## Beschreibung

Die Erfindung betrifft ein Verfahren zur Herstellung künstlicher Sauerstoffträger aus chemisch vernetzten Hämoglobinen mit verbesserten funktionellen Eigenschaften, wobei vor der Vernetzung der Hämoglobine chemisch nicht reagierende Effektoren der Sauerstoffaffinität der Hämoglobine zugesetzt werden und während der Vernetzung anwesend sind. Dies führt erfindungsgemäß zum reversiblen Schutz derjenigen konformativen Bereiche der Hämoglobin-Moleküle, die die Wechselwirkung der Hämoglobine mit Sauerstoff steuern. Dieser nicht-chemische Schutz bewirkt, dass sich die Sauerstoffbindungseigenschaften (insbesondere Affinität und Kooperativität) während einer Vernetzung der Hämoglobine modifiziert ändern.

Ein Grund, warum man native Hämoglobine chemisch verändert und modifiziert, ist die Entwicklung und Herstellung künstlicher Sauerstoffträger aus solchen Hämoglobinen. Unveränderte native Hämoglobine - insbesondere als funktionell hochwirksame molekulardisperse Form im Blutplasma gelöst - eignen sich nicht als künstliche Träger, weil sie von der Niere in strukturelle Untereinheiten zerlegt und diese schnell ausgeschieden werden, weil sie weiterhin auch die Kapillaren verlassen und weil unerwünschte Wechselwirkungen mit Plasma-Proteinen auftreten.
Die Sauerstoff-Träger wiederum will man deshalb entwickeln, weil sehr viele und weitverbreitete krankhafte Veränderungen auf einem Sauerstoffmangel von Geweben beruhen, im akuten Fall nach einem starken Blutverlust - beispielsweise nach einem Unfall oder während großer chirurgischer Eingriffe - und im chronischen Fall bei Durchblutungsstörungen, letzteres beispielsweise im Fall der sogenannten Arteriellen Verschiusskrankheit, ferner bei Herzinfarkt, bei Herzrhythmusstörungen, beim Schlaganfall, beim Niereninfarkt und anderen mehr. Da künstliche Sauerstoffträger sehr effektive Sauerstofftransporter sind, lassen sich die genannten Erkrankungen mit ihnen sehr wirksam bekämpfen.

Zur Entwicklung künstlicher Sauerstoffträger, die die genannten Nachteile nicht besitzen, hat man verschiedene gedankliche Ansätze zu verwirklichen versucht. Die wichtigsten sind
- die Mikroverkapselung von Hämoglobinlösungen in Liposomen, sogenannte Hämosomen (Ogata Y. (1994): "Characteristics of Neo Red Cells, Their Function and Safety: In Vivo Studies", *Artificial Cells, Blood Substitutes, and Immobilization Biotechnologies* 22: 875 - 881);
- kovalente intramolekulare Verknüpfungen, d. h. eine Stabilisierung der Quartärstruktur der Hämoglobine, entweder durch bifunktionelle Vernetzer (Farmer M.C., et al. (1995): "Preclinical Data and Clinical Trials with Diaspirin Cross-linked Hemoglobin", - Tsuchida E. (Ed.): *Artificial Red Cells,* John Wiley 1995: 177 - 185; Bakker J.C., et al. (1988): "Properties of Hemoglobin Interdimerically Cross-linked with NFPLP", *Biomaterials, Artificial Cells, and Immobilization Biotechnologies* 16: 635-636), oder durch gentechnische Gewinnung entsprechend veränderten Hämoglobins (Looker D. et al. (1992): "A Human Recombinant Haemoglobin Designed for Use as a Blood Substitute", *Nature* 356: 258 - 260); vgl. auch US-A 4,600,531, betreffend die Herstellung intramolekular Verknüpften Hämoglobins mit Hilfe von Fumarsaürederivaten in gegenwert nicht reagierenden Polyanionen wie z.B. Inositol hexasulfat.
- kovalentes Anknüpfen von Makromolekülen an das Hämoglobin, beispielsweise Polysaccharide, Dextrane, Hydroxyethylstärke, Inulin oder künstliche wasserlösliche Makromoleküle wie Polyethylenglykole (Xue H., Wong J. T.-F. (1994): "Preparation of Conjugated Hemoglobins", - Abelson J.N., Simon M.I. (Ed.): *Methods of Enzymology, Volume 231 B*, Academic Press 1994: 308 - 322; Tam S.C., et al. (1978): "Blood Replacement in Dogs by Dextran-Hemoglobin", *Canadian Journal of Biochemistry* 56: 981 - 984; Patentschrift DE-A 30 26 398 (1981): "Modifiziertes Hämoglobin enthaltender Blutersatz" ; Patentschrift EP-A 0 206 448 (1986): "Hemoglobin Combined with a Poly(Alkylene Oxide)"; Patentschrift US 5,234,903 (1993): "Chemically Modified Hemoglobin as an Effective, Stable, Non-immunogenic Red Blood Cell Substitute" ; Patentschrift US 5,312,808 (1994): "Fractionation of Polyalkylene Oxide-Conjugated Hemoglobin Solutions") ;
- intermolekulares Vernetzen (Gould S.A., et al. (1998): "The Clinical Development of Human Polymerized Hemoglobin", - Chang T.M.S. (Hrsg.): *Blood Substitutes: Principles, Methods, Products and Clinical Trials, Volume 2,* Karger Landes Systems 1998: 12 - 28 ; Pearce L.B., Gawryl M.S. (1998): "Overview of Preclinical and Clinical Efficacy of Biopure's HBOCs", - Chang T.M.S. (Hrsg.): *Blood Substitutes: Principles, Methods, Products and Clinical Trials, Volume* 2, Karger Landes Systems 1998: 82 - 98; Bakker J.C., et al. (1992): "Preparation and Characterization of Crosslinked and Polymerized Hemoglobin Solutions", *Biomaterials, Artificial Cells, and Immobilization Biotechnologies* 20: 233 - 241).

Die- letztgenannten künstlichen Sauerstoffträger auf der Basis vernetzter Hämoglobine besitzen gegenüber den anderen eine Reihe von Vorteilen: Ausreichend große vernetzte Hämoglobine (Hämoglobin-Polymere) verfügen über einen so geringen kolloidosmotischen Druck, dass sie nicht nur - wegen ihres so geringen eigenen kolloidosmotischen Druckes dann kombiniert mit einem Plasmaexpander - als Sauerstoff transportierendes Blutvolumensubstitut zum Ersatz fehlenden Blutes eingesetzt werden können, sondern insbesondere können sie auch - als Sauerstoff transportierendes Blutadditiv - in Blut addiert werden (Barnikol W.K.R., et al. (1996): "Hyperpolymere Hämoglobine als künstliche Sauerstoffträger. Ein innovativer Ansatz der medizinischen Entwicklung", *Therapiewoche* 46: 811 - 815). Indikationsgebiete für solche Sauerstofftransport-Additive sind die Behandlung vieler chronischer Sauerstoffmangelzustände, wie sie oben erwähnt wurden. Die Behandlung mit einem Additiv ist immer auch ohne einen vorherigen Blutverlust möglich; dagegen sind sämtliche genannten Sauerstoff transportierenden (Blut-) Volumensubstitute ausschließlich zur Behandlung akuter Sauerstoffmangelzustände nach Blutverlusten geeignet. Hämoglobine mit hohem Vernetzungsgrad besitzen darüber hinaus den Vorteil besonders langer intravasaler Verweildauer. Weiterhin muß nach ihrer Gabe nicht mit Blutdruckerhöhungen gerechnet werden, da sie auf Grund ihrer Größe die Blutgefäße nicht verlassen und somit nicht als Konstriktoren der Gefäßmuskulatur wirken können.

Die regelrechte Versorgung der Gewebe mit Sauerstoff durch das natürliche Hämoglobin, welches sich in den roten Blutzellen befindet, beruht wesentlich auf der besonderen Sauerstoff-Bindungscharakteristik des Hämoglobins als S-förmige Bindungskurve. Diese Bindungscharakteristik lässt sich mit zwei Größen kennzeichnen, nämlich mit dem sogenannten Halbsättigungsdruck des Sauerstoffs (P50) als Maß der mittleren Sauerstoffaffinität des Hämoglobins und dem HILLschen Index (n50) als Maß der homotropen Wechselwirkungen, der sogenannten Kooperativität der Sauerstoffbindungsstellen.
Beide Größen bestimmen in entscheidender Weise, wie effektiv in der Lunge der Sauerstoff aus der Luft ins Blut aufgenommen und wie gut der Sauerstoff in den Kapillaren wieder vom Blut an die Gewebe abgegeben werden kann. Normalerweise hat das in den roten Blutzellen abgepackte Hämoglobin im menschlichen Blut einen P50-Wert von etwa 25 Torr und den hohen n50-Wert von 2,6; die entsprechenden Werte des frei gelösten Hämoglobins des Menschen und des Schweins betragen unter physiologischen Bedingungen etwa 16 Torr und ebenfalls 2,6. Es ist seit langem bekannt, dass natürlicherweise bei Säugern in deren roten Blutzellen die regelrechte Sauerstoff-Bindungs-Charakteristik durch kleine effektorische Moleküle bewirkt wird, die assoziativ (also nicht-kovalent) an das Hämoglobin binden können. Bei Mensch und Schwein handelt es sich hierbei um 2,3-Bisphosphoglycerat (DPG). Dieser Effektor bindet nicht-kovalent in der sogenannten zentralen Kavität des Hämoglobinmoleküls, welche durch die vier pseudo-tetraedisch angeordneten, globulären Untereinheiten dieses Moleküls gebildet wird; er tut dies besonders fest im desoxygenierten Zustand des Hämoglobins. Neben dem natürlichen kennt man auch artfremde und künstliche Effektoren, welche die Bindungseigenschaften des Human-Hämoglobins ebenfalls stark beeinflussen, beispielsweise Inositolhexaphosphat, Inositolhexasulfat und Mellitsäure (W.K.R. Barnikol, O. Burkhard (1983): "Die Feinstruktur als Hilfsmittel zum Studium pharmakologischer Wirkungen auf die Sauerstoff-Hämoglobin-Bindung. Hämoglobin als Puffer des Sauerstoffpartialdruckes", *Funktionelle Biologie und Medizin* 2: 245 - 249). Offensichtlich und selbstverständlich binden sich solche Effektoren spezifisch an die Sauerstoff-abhängigen Bereiche des Hämoglobin-Moleküls.

Für molekular-disperse künstliche Sauerstoffträger kommt es, unabhängig von der gewählten Entwicklungsstrategie, darauf an, durch die notwendigen chemischen Modifikationen die mittlere Affinität auf einen bestimmten gewünschten Wert einstellen zu können und die natürliche Kooperativität möglichst auf ihrem hohen Wert zu erhalten.

Die Herstellung künstlicher Sauerstoffträger auf der Basis vernetzter Hämoglobine erfordert zunächst die kovalente Vernetzung des Hämoglobins zu großen Molekülen. Darüber hinaus können diese dann mit inerten und bio-verträglichen Molekülen kovalent verknüpft werden, um unerwünschte Wechselwirkungen mit Plasmaproteinen zu vermeiden. Sowohl die Vernetzung, als auch kovalente chemische Anknüpfungen erfolgen bevorzugt an Aminogruppen der Hämoglobin-Moleküle. Führt man die genannten chemischen Reaktionen mit Hämoglobinen durch, so finden sich in aller Regel unerwünschte Veränderungen der Sauerstoffaffinität, vor allem aber ein Verlust der Kooperativität (eine Verringerung des n50-Wertes), beispielsweise bei Vernetzung des Humanhämoglobins mit Divinylsulfon (H. Pötzschke, St. Guth, W.K.R. Barnikol: "Divinylsulfone-Crosslinked Hyperpolymeric Human Haemoglobin as an Artificial Oxygen Carrier in Anaesthetized Spontaneously Breathing Rats", *Advances in Experimental Biology and Medicine Vol. 345,* Plenum Press, New York 1994: 205 - 214), insbesondere dann, wenn man einen hohen Vernetzungsgrad möglichst ohne monomeres Hämoglobin erreichen möchte.

Die Aufgabe der vorliegenden Erfindung ist es, eine Methode zu entwickeln, durch welche die bei einer Vernetzung von Hämoglobinmolekülen entstehenden Änderungen der Sauerstoff-Bindungseigenschaften bezüglich der Affinität zum Sauerstoff sowie dem Ausmaß der homontropen Kooperativität der Sauerstoffbindungsstellen der vernetzten Hämoglobine verhindert oder in gewünschter Weise modifiziert werden.

Erfindungsgemäß wird die Aufgabe gelöst, indem man vor der Vernetzung der Hämoglobine einen chemisch nicht reagierenden Effektor der Sauerstoffaffinität der Hämoglobine zur Reaktionslösung gibt und diesen während der Vernetzungsreaktion dort belässt.

Überraschenderweise zeigte sich, dass bekannte Effektoren der Sauerstoffbindung der Hämoglobine sich auch dazu nutzen lassen, um diese Hämoglobine während Vernetzungen, beispielsweise mit Glutardialdehyd, reversibel und wirkungsvoll gegen eine Änderung der Sauerstoffaffinität durch den Vernetzer zu schützen, vor allem, die Verminderung der Kooperativität entscheidend zu verringern. Dies ist besonders wichtig für so reaktive und effektive Vernetzer, wie der genannte Glutardialdehyd.

Als Schutzeffektoren werden erfindungsgemäß Inositolhexaphosphat, Inositolhexasulfat, Mellitsäure und besonders 2,3-Bisphosphoglycerat, die bevorzugt in Mengen von 1 bis 20, insbesondere 1 bis 10 mol und ganz besonders 1 bis 3, insbesondere 2 mol pro Mol unvernetztes Hämoglobin zugesetzt.

Die Vernetzer werden ausgewählt aus Butadiendiepoxid, Divinylsulfon, einem Diisocyanat, insbesondere Hexamethylendiisocyanat, Zyklohexyldiisocyanat und 2,5-Bisisocyanatobenzolsulfonsäure, einem Di-N-Hydroxysuccinimidylester, einem Diimidoester, oder einem Dialdehyd, insbesondere Glyoxal, dem analog reagierenden Glykolaldehyd, oder Glutardialdehyd.

Als Hämoglobin-Ausgangsmaterial für die erfindungsgemäße Lehre eignet sich monomeres, natives oder chemisch modifiziertes Hämoglobin vom Menschen, vom Schwein, oder vom Rind, bevorzugt ist humanes und insbesondere Schweine-Hämoglobin. Die chemische Modifikation des Hämoglobins kann beispielsweise in einer kovalenten Anknüpfung bestimmter niedermolekularer Stoffe bestehen, wie Effektoren der Sauerstoffaffinität, z. B. Pyridoxal-5'-Phosphat oder 2-Nor-2-Formyl-Pyridoxal-5'-Phosphat (wie in Kothe et al. (1985), Surgery, Gynecology & Obstetrics 161: 563-569 oder Van der Plas et al. (1987), Transfusion 27: 425-430 und (1988), Transfusion 28: 525-530 beschrieben, weitere Zitate in: Rudolph A. S. et al. (Hrsg.): *Red Blood Cell Substitutes: Basic Principles and Clinical Applications,* Marcel Dekker, New York u. a. 1998; Tsuchida E. (Hrsg.): *Blood Substitutes: Present and Future Perspectives,* Elsevier Science, Amsterdam 1998; Chang T. M. S. (Autor bzw. Hrsg.): *Blood Substitutes: Principles, Methods, Products and Clinical Trials, Volume 1* und ~ *Volume 2,* Karger Landes, Basel u. a. 1997 und 1998, vgl. auch EP 0 528 841, dort wird die Pyridoxylierung von Hämoglobin beschrieben). Bevorzugt werden diese Moleküle vor der erfindungsgemäßen Vernetzung angeknüpft.
Alernativ oder zusätzlich kann eine Modifikation mit Molekülen erfolgen, welche die Verträglichkeit der entstehenden künstlichen Sauerstoffträger mit Plasma verbessern. Hierzu gehören z. B. Polyethylenglykole (Übersicht in: Harris J. M. (Hrsg.): *Poly(Ethylene Glycol) Chemistry: Biotechnical and Biomedical Applications,* Plenum, New York u. a. 1992).Die Umsetzung hiermit wird nachfolgend beschrieben.
Das Hämoglobin kann darüberhinaus bevorzugt auf bekannte Weise desoxygeniert oder karbonyliert werden.
Bevorzugt ist natives, insbesondere monomeres Hämoglobin vom Menschen und insbesondere vom Schwein.

Vernetzungen monomeren Hämoglobins mit etlichen Vernetzern sind bekannt und in der Literatur vielfach beschrieben, beispielhaft seien angeführt:
Die Patentschriften US 4,001,200 und US 4,001,401 betreffen vernetzte Hämoglobine sowie ihren Einsatz als Blutersatz und Plasmaexpander. Die Molekulargewichte (Molaren Massen) dieser vernetzten Hämoglobine betragen von 65 000 bis 1 000 000 g/mol. Sie können hergestellt werden mittels einer Vielzahl genannter verknüpfender Agenzien, wie beispielsweise Divinylsulfon, Epichlorhydrin, Butadiendiepoxid, Hexamethylendiisocyanat, den Dialdehyden Glyoxal und Glutardialdehyd sowie den Diimidoestern Dimethylsuberimidat, Dimethylmalonimidat und Dimethyladipimidat.
Die Patentschrift DE 24 49 885 betrifft (u. a.) auch vernetzte Hämoglobine, die durch Umsetzung nicht-vernetzter Hämoglobine mit etlichen Dialdehyden, beispielsweise Malondialdehyd, Succindialdehyd, Glutardialdehyd, Adipindialdehyd und Suberdialdehyd hergestellt werden können.
Die Patentschrift US 4,357,636 beschreibt die Herstellung verschiedener vernetzter Hämoglobine durch Umsetzung von Hämoglobin mit etlichen Dialdehyden und
Polyaldehyden, beispielsweise einfachen wie Glutardialdehyd und Glyoxal, aber auch mit strukturell komplexeren, die durch oxidative Ringöffnung der zyklischen Halbazetal- und
Halbketalstrukturen der Zuckermoleküle in Monosacchariden und Oligosacchariden sowie Derivaten dieser entstehen.
Die Patentschrift US 5,439,882 handelt von vernetzten Hämoglobinen, die durch Umsetzung mit den Dialdehyden o-Adenosin und o-ATP, entstanden durch ringöffnende Oxidation der Ribose in Adenosin und in Adenosintriphosphat, hergestellt sind. Diese vernetzten Hämoglobine besitzen Molekulargewichte von 65000 bis 390000 g/mol.
Die Patentschrift EP 0 201 618 betrifft ein Verfahren, aus hoch konzentrierten Lösungen monomerer Hämoglobine extrem hochmolekulare lösliche Hämoglobinpolymere, sogenannte Hyperpolymere (Molekulargewichte 65000 bis 15000000), herzustellen.

Die beschriebenen Verfahren können gemäß der vorliegenden Methode angewendet werden, wobei erfindungsgemäß unmittelbar vor der Vernetzungsreaktion der nicht reaktive Vernetzer in der angegebenen Menge zugesetzt wird.

Prinzipiell erfolgt die Vernetzung des Hämoglobins unter Verwendung eines geeigneten poly- oder bifunktionellen Vernetzers für Proteine, bevorzugt werden bifunktionelle. Vernetzer wie Butandiepoxid, Divinylsulfon, ein Diisocyanat, insbesondere Hexamethylendiisocyanat, Zyklohexyldiisocyanat oder 2,5-Bisisocyanatobenzolsulfonsäure, ein Di-N-Hydroxysuccinimidylester, ein Diimidoester, oder ein Dialdehyd, insbesondere Glyoxal, der analog reagierenden Glykolaldehyd, oder Glutardialdehyd.
Ganz besonders bevorzugt erfolgt die Vernetzung mit Glutardialdehyd, wie z. B. in Pötzschke H. und Barnikol W. (1992), *Biomaterials, Artificial Cells, and Immobilization Biotechnology* 20: 287 - 291, oder wie in den nachfolgenden Beispielen beschrieben.
Es werden, je nach verwendetem Vernetzer, molare Verhältnisse der Vernetzer- bezogen auf monomeres Hämoglobin - von 3- bis 60-fach, bevorzugt 6- bis 35-fach, eingesetzt, beispielsweise bevorzugt zwischen einem 7- und 10-fachen molaren Überschuss des Glutardialdehyds. Chemisch nicht stabile Verknüpfungen, insbesondere die Schiffschen Basen, die bei der Reaktion von funktionellen Aldehydgruppen mit Aminogruppen der Hämoglobine entstehen, werden in bekannter Weise reduktiv durch Reaktion mit geeigneten Reduktionsmitteln, wie z. B. Natriumborhydrid, in einem hinreichenden molaren Überschuss bezogen auf monomeres Hämoglobin, bevorzugt 2- bis 100-fach, insbesondere bevorzugt 5- bis 20-fach, unter geeigneten bekannten Bedingungen stabilisiert.

Die Durchführung der Vernetzungen wie -auch der Derivatisierungsreaktionen- richtet sich nach den Erfordernissen der gewählten chemischen Reaktionen: Native und modifizierte Hämoglobine sind Polyelektrolyte und befinden sich deshalb zur Reaktion mit dem Vernetzungsmittel in wässrigen Elektrolyten, die z. B. Kochsalz oder/und Natriumhydrogenkarbonat enthalten, mit Ionenkonzentrationen bis 300 mmol/L, vorzugsweise zwischen 50 und 200 mmol/L. Die Reaktionstemperatur zur Vernetzung der Hämoglobine beträgt 4 bis 65, vorzugsweise 3 bis 30 und insbesondere 4 bis 10 °C, die Protonenaktivität in den Lösungen, ausgedrückt als pH-Werte, betragen zwischen 5 und 11, bevorzugt zwischen 6 und 9 und insbesondere zwischen 6,5 und 8, sie können in bekannter Weise, z. B. mit Milchsäure oder Natronlauge, auf den gewünschten Wert eingestellt werden. Der Effektor wird der Reaktionslösung hinzu gesetzt, welche das noch unvernetzte Hämoglobin in einer Konzentration von 10 bis 420, insbesondere von 150 bis 400 g/L enthält.
Das Hämoglobin kann, z. B. durch Überströmen der Lösung mit Stickstoff oder anderen sauerstoffreien Inertgasen, desoxygeniert werden. Anschliessend wird in bekannter Weise das Vernetzungsreagenz in einem geeigneten molaren Verhältnis bezogen auf monomeres Hämoglobin, hinzugefügt. Die Reaktionszeiten betragen, je nach der gewählten speziellen Reaktion, auch in Abhängigkeit von Temperatur, pH-Wert, lonenkonzentration u. s. w., zwischen wenigen Minuten und bis zu 3 Tagen, bevorzugt weniger als 5 Stunden und ganz bevorzugt weniger als 2 Stunden.
Der Überschuss an Vernetzungsmittel kann dann beispielsweise chemisch durch Reaktion mit geeigneten Reduktionsmitteln, oder durch physikalische Methoden entfernt werden.

Die so hergestellten Hämoglobine können gegebenenfalls wie erwähnt anschließend weiter chemisch modifiziert, z. B. mit Polyalkylenoxiden verknüpft werden.
Kovalente Anknüpfungen von Polyalkylenoxiden an Proteine, insbesondere auch an unvernetztes Hämoglobin, sind etliche bekannt und in der Literatur beschrieben (den Stand der Technik beschreibt umfassend: Harris J. M. (Hrsg.): *Poly (Ethylene Glycol) Chemistry: Biotechnical and Biomedical Applications,* Plenum, New York u. a. 1992). Bei sehr vielen dieser Verfahren erfolgt die Anknüpfung des Polyalkylenoxids über eine molekulare Brücke (*"spacer"*), die beispielsweise ein bifunktioneller Verknüpfer schafft. Streng betrachtet wird in diesen Fällen also ein. Verknüpfungsprodukt eines Polyalkylenoxids mit einem Verknüpfungsreagenz an das Protein geknüpft.
Die Anbindung von Polyalkylenoxiden an Proteine ist bekannt (z. B.: Patent US 4,179,337 (1979): "Non-immunogenic Polypeptides"), speziell auch an Hämoglobine, namentlich auch an künstliche Sauerstoffträger auf der Basis modifizierter Hämoglobine, (Patentschriften US 5,478,805 (1995): "Fractionation of Polyalkylene Oxide-Conjugated Hemoglobin Solution", US 5,386,014 (1995): "Chemically Modified Hemoglobin as an Erective, Stable, Non-immunogenic Red Blood Cell Substitute", EP-A 0 206 448 (1986): "Hemoglobin Combined with a Poly (Alkylene Oxide)", EP-A 0 067 029 (1982): "Oxygen Carrier"). Die Anknüpfung von Polyalkylenoxiden an künstliche Sauerstoffträger auf der Basis modifizierter Hämoglobine wurde nach der bekannten Literatur allerdings nie an einem vernetzten Hämoglobin vorgenommen, und diente immer dem Erreichen gänzlich anders gearteter Ziele, beispielsweise einer Verlängerung der intravasalen Verweildauer, oder auch zur Verminderung der immunogenen Potenz der künstlichen Sauerstoffträger.

Zur kovalenten Anknüpfung der Polyalkylenoxide werden bevorzugt solche Derivate der Polyalkylenoxide verwendet, die ein verknüpfendes Agens mit einer funktionellen Gruppe bereits kovalent gebunden enthalten, welche eine direkte chemische Reaktion mit Amino-, Alkohol-, oder Sulfhydryl-Gruppen der Hämoglobine unter Bildung kovalenter Anknüpfungen der Polyalkylenoxide ergeben - beispielsweise Polyalkylenoxide mit reaktiven N-Hydroxysuccinimidylester-, Epoxid- (Glycidylether-), Aldehyd-, Isocyanat-, Vinylsulfon-, Jodazetamid-, Imidazolylformat-, Tresylatgruppen, u. a. Viele solche monofunktionell aktivierte Polyethylenglykole sind kommerziell erhältlich, z. B. die genannten, und zwar mit Molekulargewichten zwischen etwa 500 und 5 000 g/mol.

Bevorzugt werden Derivate eines Polyalkylenoxids, insbesondere ausgewählt aus Polyethylenoxid, Polypropylenoxid oder Copolymeren hiervon, eingesetzt. Besonders bevorzugt sind Verknüpfungsprodukte des Polyalkylenoxids, insbesondere der genannten, mit einem eine terminale Hydroxygruppe maskierenden Molekül, insbesondere als Ether, Ester, Esteramid mit kurzkettigen (C₁ . C₅) aliphatischen organischen Rest eingesetzt.

Alternativ können nicht-aktive Polyaikylenoxide in jeder weiteren geeigneten Weise zunächst chemisch aktiviert oder, eventuell nach einer zusätzlich notwendigen Derivatisierung, durch chemische Verknüpfungsagenzien mit dem Hämoglobin verknüpft werden, beispielsweise mittels chemischer Reaktion mit Bromcyan, einem Karbodiimid wie beispielsweise 1-Ethyl-3-(3-Dimethylaminopropyl)karbodiimid oder N,N'-Dizykiohexylkarbodiimid, Cyanurchlorid (mit diesem aktivierte Polyethylenglykole, 4,6-Dichlor-s-triazin-Polyethylenglykole, sind ebenfalls kommerziell erhältlich), oder anderen, bekannten Verknüpfungsagenzien wie beispielsweise 2,2'-Dichlorbenzidin, p,p'-Difluor-m,m'-dinitrodiphenylsulfon, 2,4-Dichlornitrobenzol und weiteren (Überblick in: Harris J. M. (Hrsg.): *Poly (Ethylene Glycol) Chemistry: Biotechnical and Biomedical Applications,* Plenum, New York u. a. 1992).
Als Polyalkylenoxide eignen sich besonders Polyethylenoxide (Polyethylenglykole), Polypropylenoxide (Polypropylenglykole), sowie Kopolymere (Mischpolymere) aus Ethylenoxid und Propylenoxid,insbesondere, wie erwähnt, gewisse Derivate dieser, wie eine OH-Gruppe maskierende Verbindungen, beispielsweise (Mono-)Ether mit einem kurzkettigen Alkohol, vorzugsweise mit 1 bis 5 Kohlenstoffatomen, wie Monomethylether, Monoethylether, Monopropylether, u. s. w., (Mono-)Ester mit kurzkettigen Karbonsäuren, vorzugsweise mit 1 bis 5 Kohlenstoffatomen, wie Monomethylester, Monomethylester, Monopropylester, u. s. w. und Dehydratisierungsprodukte mit einem aliphatischen Amin mit 1 bis 5 Kohlenstoffatomen, wie Monomethylamin, Monoethylamin, Monopropylamin, u. s. w. mit dem oben angegebenen. Besonders bevorzugt sind Polyethylenglykole und deren genannte Derivate.
Das Molekulargewicht der verwendeten Polyalkylenoxide beträgt bevorzugt zwischen 200 und 5000 g/mol, insbesondere zwischen 500 und 2000 g/mol.
Diese werden vorzugsweise in einer Menge von 1-40, insbesondere 4-15 Mol/Mol Hämoglobin eingesetzt.

Die Durchführung der Anknüpfungen bei der erfindungsgemäßen Vorgehensweise ist analog, wie oben beschrieben.
Das Hämoglobin kann somit mit Hilfe der bekannten Methoden mit Polyalkylenoxid verknüpft werden, wie oben beschrieben, beispielsweise durch direkte Kombination mit Hilfe eines Kondensationsmittels, wie Bromcyan, oder mit Hilfe eines Vernetzungsreagenz, wie beispielsweise Cyanurchlorid (vgl. DE-OS 30 26 398), oder durch Reaktion mit einem aktivierten Polyalkylenoxid, beispielsweise einem N-Hydroxysuccinimidylester eines Polyalkylenoxidderivates. Auf diese Weise werden mindestens 1, insbesondere 1 bis 40 und vorzugsweise 4 bis 15 Moleküle des erfindungsgemäß verwendeten Polyalkylenoxids je Molekül monomeren Hämoglobins verknüpft.

Beispielhaft können folgende Verfahren für die Anknüpfung der Polyalkylenoxide angewendet werden, wobei deren strukturelle Integrität erhalten bleibt:
(1) (Nicht aktiviertes) Polyethylenglykol wird mit der 2- bis 5-fachen molaren Menge, vorzugsweise der 3-fachen molaren Menge an Bromcyan bei einem pH-Wert von 9 bis 10 umgesetzt. Das restliche Bromcyan wird durch Gelfiltration, Dialyse etc. aus dem Reaktionsgemisch entfernt und das Produkt wird dann mit einer erforderlichen, z. B. 0,1- bis 0,002-fachen, vorzugsweise der 0,02- bis 0,01-fachen molaren Menge an Hämoglobin bei pH 7 bis 9, vorzugsweise 7,5 bis 8, in wässriger Lösung umgesetzt (vgl. DE-OS 3 026 398);
(2) Polyethylenglykol wird in Benzol gegeben, welches eine überschüssige Menge an Natriumkarbonat enthält, und dann mit der 2- bis 5-fachen molaren Menge, vorzugsweise der 3- bis 4-fachen molaren Menge an Cyanursäurechlorid umgesetzt. Das Reaktionsprodukt, Polyethylglykol-4,6-dichlor-s-triazin, wird abgetrennt und mit der gewünschten Menge, z. B. 1 bis 0,002 mol, vorzugsweise 0,1 bis 0,01 mol, bezogen auf ein Mol des vorstehend genannten Reaktionsprodukts, an Hämoglobin in einer Pufferlösung mit einem pH-Wert von 8 bis 9,5 umgesetzt (vgl. DE-OS 30 26 398);
(3) Aktiviertes Polyalkylenoxid, beispielsweise ein N-Hydroxysuccinimidylester eines Polyalkylenoxids, wird in einem 1- bis 40-fachen molaren Überschuss bezogen auf monomeres Hämoglobin zu einer wässrigen Lösung mit einem pH zwischen 7 und 10 eines mit dem Polyalkylenoxid zu verknüpfenden Hämoglobins gegeben und reagieren lassen.

Die vorstehend erläuterten Methoden lassen sich auch im Fall der anderen erfindungsgemäss verwendeten Polymeren anwenden.

Die chemische Anbindung der Polyalkylenoxide an die künstlichen Sauerstoffträger aus vernetzten Hämoglobinen kann im Verlauf der Herstellung der erfindungsgemäßen Hämoglobin-Derivate zu drei Zeiten erfolgen:
i) Im ersten Fall wird das Polyalkylenoxid-Derivat an die hochreinen, nativen oder modifizierten Hämoglobine (Hämoglobin-Monomere) gebunden, im Anschluss daran erfolgt dann die Vernetzung der Hämoglobine mit einem insbesondere bifunktionellen Vernetzer.
ii) im zweiten Fall werden Polyalkylenoxid-Derivate an das bereits synthetisierte vernetzte Hämoglobin angekoppelt, d.h. im Anschluss an die Umsetzung der hochreinen, nativen oder mit Effektoren modifizierten Hämoglobin-Monomere mit einem bifunktionellen Vernetzer.
iii) Im dritten Fall schließlich kann eine kovalente Anbindung von Polyalkylenoxid-Derivaten sowohl an die Hämoglobin-Monomeren vor deren Vernetzung, als auch zusätzlich danach, im weiteren Verlauf der Herstellung, an das vernetzte Hämoglobin durchgeführt werden.

Wie erwähnt, erfolgt jeweils vor der Vernetzung der erfindungsgemäße Zusatz an Effektor. Bevorzugt erfolgt die Modifikation mit einem Polyalkylenoxid, insbesondere Polyethylenoxid oder Derivaten hiervon wie o.g., nach der erfindungsgemäßen Vernetzung.

Die erhaltenen vernetzten Hämoglobine können dann auf geeignete bekannte Weise, beispielsweise durch Zentrifugation, Filtration oder Ultrafiltration, oder chromatographisch (z. B. durch präparative Volumenausschluss-Chromatographie, wie z.B. an Sephadex G-25 Gel) oder wie in den obengenannten Druckschriften oder in Curling J.M.: Methods of Plasma Protein Fractionation, Academic Press, London, 1980, oder EP-A 0 854 151, EP-A 95 107 280 beschrieben, gereinigt und nachfolgend zu einer pharmazeutischen Zubereitung weiterverarbeitet werden.

Vorzugsweise wird monomeres Hämoglobin, bevorzugt im desoxygenierten Zustand, in einem wässrigen Elektrolyten (der z. B. Natriuhydrogenkarbonat oder Natriumchlorid oder Natriumlaktat oder mehrere dieser enthält), zunächst mit einem der genannten Effektoren in den angegebenen Mengen gemischt und nachfolgend vernetzt, beispielsweise mit den genannten bifunktionellen Vernetzern, insbesondere mit einem 7- bis 10-fachen molaren Überschuss an Glutardialdehyd. Überschüssiger Glutardialdehyd wird auf bekannte Weise mit Natriumborhydrid entfernt, z. B. durch Zusatz eines 2- bis 100-fachen, insbesondere eines 5- bis 20-fachen molaren Überschusses, bezogen wiederum auf das monomere Hämoglobin. Die so erhaltenen vernetzten Hämoglobine können dann nachfolgend z. B. durch Dialyse, Zentrifugation, Klär- und Ultrafiltration, Fällung, beispielsweise mit Polyethylenoxid, präparative chromatographische Methoden, wie z. B. durch Gelpermeationschromatographie, aufgearbeitet und auch zu einer pharmazeutischen Zubereitung als künstlicher Sauerstoffträger weiter verarbeitet werden oder auch noch mit Polyalkylenoxid umgesetzt und aufgearbeitet werden und sodann zu einer pharmazeutischen Zubereitung verarbeitet werden.

Auf diese Weise wird als Produkt ein vernetztes Hämoglobin erhalten, welches durch die Anwesenheit des nicht reaktiven Effektors während der Vernetzung besonders günstige Sauerstoffaffinitätseigenschaften durch das Vermeiden oder Verringern eines Verlusts an Kooperativität aufweist. Dieser erzielbare Vorteil der erfindungsgemäßen Lehre war im Hinblick auf den Stand der Technik überraschend, da in diesem eine Behandlung mit nicht-reaktiven Effektoren nicht bekannt ist und eine Einflussnahme solcher nicht reaktiven Moleküle bei den chemischen Umsetzungen insbesondere mit hoch reaktiven Vernetzungsreagentien nicht erwartet werden konnte. Die erfindungsgemäßen vernetzten Hämoglobine weisen einen äußerst günstigen unerwarteten Vorteil auf, nämlich eine Verminderung des Verlustes der Kooperativiät der Sauerstoffbindung, verglichen mit ganz analog aber ohne die erfindungsgemäße Verfahrensweise hergestellten vernetzten Hämoglobinen.

Werden an die vernetzten Hämoglobine außerdem Polyalkylenoxide kovalent angeknüpft, kann zusätzlich - auch unter extremen physiologischen Bedingungen insbesondere des pH-Wertes - eine deutlich verbesserte Plasmaverträglichkeit erzielt werden. Die Verträglichkeit ist dabei unabhängig von der Art und dem Molekulargewicht des Hämoglobins, vom verwendeten Vernetzer, Effektoren oder dem eingesetzten Polyalkylenoxid.

Die erfindungsgemäß hergestellten Hämoglobin-Derivate können als solche oder in Form geeigneter, z.B. pharmazeutischer Zubereitungen als künstliche Sauerstoffträger intravasal als Pharmazeutikum oder für biomedizinische Zwecke, als Ersatz des Blutes zur Behandlung eines Blutvolumenmangels, als Zusatz zum Blut zur Behandlung pathogener Sauerstoffmangelzustände, oder als eine Nährlösung, im menschlichen oder tierischen Organismus, in Organen oder in biotechnischen Anwendungen, verwendet werden. Zur Herstellung der zu verabreichenden Produkte werden die erfindungsgemäßen Hämoglobinprodukte in geeigneten Medien, wie Infusionslösungen, beispielsweise in wässriger Kochsalz- oder Glukoselösung, beide vorzugsweise in dem Blutplasma isotonischen Konzentrationen, gelöst.

Besonders bevorzugte Ausgestaltungen der Erfindung werden nachfolgend zunächst anhand eines allgemeinen Herstellungsverfahrens und nachfolgender Beispiele näher erläutert:

Schweine-, Human- oder Rinderhämoglobin, nativ oder nach einer vorangegangenen chemischen Modifikation beispielsweise mit einem kovalent bindenden Effektor der Sauerstoffaffinität und/oder einem kovalent bindenden Polyalkylenoxid zur Verbesserung der Verträglichkeit mit Plasmaproteinen, mit einer Konzentration zwischen 10 und 420 g/L, bevorzugt zwischen 150 und 400 g/L, ist in einer wässrigen Natriumhydrogenkarbonat-Lösung gelöst, diese besitzt eine Konzentration zwischen 40 und 100 mmol/L und eine Temperatur aus dem Bereich zwischen 3 und 30 °C. Durch Überströmen mit reinem Stickstoff und Rühren dieser Hämagfobinlösung erfolgt eine Desoxygenierung des Hämoglobins. Der pH der Lösung wird mit Milchsäure oder Natronlauge (einer Konzentration zwischen 0,1 und 1 mol/L) auf einen Wert zwischen 6 und 9, bevorzugt zwischen 6,5 und 8 titriert. Nun erfolgt die Zugabe von 1 bis 10, bevorzugt 1 bis 3 mol des Effektors pro Mol zu vernetzendes, monomeres Hämoglobin, insbesondere bevorzugt als Effektor ist 2,3-Bisphosphoglycerat. Anschliessend wird die Reaktion des Hämoglobins mit einem bifunktionellen Vernetzer, ausgesucht aus Butadiendiepoxid, Divinylsulfon, einem Diisocyanat, insbesondere Hexamethylendiisocyanat, Zyklohexyldiisocyanat und 2,5-Bisisocyanatobenzolsulfonsäure, einem Di-N-Hydroxysuccinimidylester, einem Diimidoester, oder einem Dialdehyd, insbesondere Glyoxal, dem analog reagierenden Glykolaldehyd, und ganz besonders bevorzugt Glutardialdehyd vorgenommen. Das molare Verhältnis des Vernetzers zum monomeren Hämoglobin beträgt zwischen 3 und 60, bevorzugt zwischen 6 und 35 mol pro Mol des monomeren Hämoglobin. Nach Vernetzung mit einem der genannten Dialdehyde werden die entstandenen Schiffschen Basen mit Natriumborhydrid, in einem molaren Verhältnis zum monomeren Hämoglobin zwischen 2 und 100, bevorzugt zwischen 5 und 20, reduziert. Diese Reduktion erfolgt bei einem pH zwischen 7,5 und 10, bevorzugt zwischen 8 und 9; dieser pH-Wert wird, wie oben beschrieben, mit Natronlauge oder Milchsäure eingestellt.
Nun können, nach erneuter Einstellung der pH-Werte zwischen 7,5 und 10 (mit Natronlauge oder Milchsäure), die vernetzten Hämoglobine mit einem Polyalkylenoxid-Derivatekovalent verknüpft werden, indem diese in einem molaren Verhältnis von 1 bis 40, bevorzugt zwischen 4 und 15 zum monomeren Hämoglobin dem Reaktionsgemisch zugegeben werden. Die Polyalkylenoxide können zur Reaktion insbesondere mit Aminogruppen der Hämoglobine bereits monofunktionell aktiviert sein, oder aktiviert oder passiv angeknüpft werden. Ohne die bevorzugte Polyalkylenoxid-Anknüpfung erfolgt direkt die weitere Aufarbeitung.

Nachfolgend wird die bevorzugte spezielle Verfahrensweise, die auch den anschließenden Ausführungsbeispielen 1 bis 3 zu grunde liegt, erläutert:

Schweinehämoglobin oder Human-Hämoglobin, das mit einer Konzentration bevorzugt zwischen 150 und 400 g/L in einem wässrigen Hydrogenkarbonat (NaHCO₃) -Elektrolyten einer Konzentration bevorzugt zwischen 40 und 100 mmol/L und einer Temperatur von 3 bis 30°C gelöst ist, wird durch Rühren und Überströmen von Stickstoff zunächst desoxygeniert, dann gibt man bevorzugt zwischen 2 und 6 mol Natrium-Ascorbat pro Mol Hämoglobin zu und titriert die Lösung mit Milchsäure auf einen pH-Wert bevorzugt zwischen 6,5 und 8. Bei dem so eingestellten pH-Wert erfolgt nun (siehe Beispiele 2 und 3) die Zugabe eines der oben genannten Effektoren der Sauerstoffaffinität in der angegebenen Menge, bevorzugt 1, 2 oder 3 mol pro Mol monomeren Hämoglobins. Darauf erfolgt die Vernetzung durch Zugabe insbesondere von Glutardialdehyd im molaren Verhältnis die Vernetzung durch Zugabe insbesonder von Glutardialdehyd im molaren Verhältnis bevorzugt zwischen 7 und 10 mol pro Mol monomeren Hämoglobins. Nach nochmaliger Titration der Lösung mit Natronlauge (NaOH) auf einen pH-Wert bevorzugt zwischen 8 und 9 erfolgt eine Reduktion der gebildeten Schiffschen Basen mit Natriumborhydrid, das in einem molaren Verhältnis bevorzugt zwischen 5 und 20 mol pro Mol monomeren Hämoglobins zugegeben wird. Mit einer weiteren Titration mit Natronlauge oder Milchsäure wird der pH-Wert in den Bereich bevorzugt zwischen 8 und 9 gebracht und es erfolgt eine Zugabe von aktiviertem Polyethylenglykol, beispielsweise einem N-Hydroxy-Succinimidyl-Derivat, mit einem Molekulargewicht von 300 bis 5000, bevorzugt zwischen 500 und 2000 g/mol, in einem molaren Verhältnis zwischen 1 und 15 mol pro Mol monomeren Hämoglobins. Danach wird das entstandene vernetzte Hämoglobin durch Überströmen von reinem Sauerstoff und Rühren zügig mit diesem ligandiert. Zur Charakterisierung der Sauerstoffbindungseigenschaften bei physiologischen Bedingungen wird nun das Lösungsmittel mitsamt allen darin enthaltenen unverbrauchten Reaktanden und

Reaktionsprodukten, beispielsweise mit Hilfe einer Volumenausschluss-Chromatographie oder einer Ultrafiltration, gegen einen der Plasmaflüssigkeit entsprechenden wässrigen Elektrolyten ausgetauscht; dieser enthält 125 mM Natriumchlorid; 4,5 mM Kaliumchlorid und 20 mM Natriumhydrogenkarbonat.

Die Erfindung wird anhand der folgenden Beispiele näher erläutert. Hierbei dient Beispiel 1 (kein Zusatz nicht-reaktiver Effektoren der Sauerstoffaffinität zur Vernetzung des Hämoglobins) als Vergleich, die Beispiele 2 und 3 repräsentieren die Erfindung und demonstrieren die erzielbaren Verbesserungen.

### Anwendungsbeispiel 1 (Vergleich für 22°C)

### Herstellung eines vernetzten Hämoglobins ohne den erfindungsgemäßen Zusatz nicht-reaktiver Effektoren der Sauerstoffaffinität während der Vernetzung des Hämoglobins bei 22°C

Eine 35%ige Lösung von Schweinehämoglobin (Hb) in 50 mM NaHCO₃ wurde bei 22 °C durch Rühren und Überströmen von Stickstoff desoxygeniert. Danach erfolgte eine Zugabe von Natrium-Ascorbat (4 mol/mol Hb). Die Lösung wurde dann mit Milchsäure auf einen pH-Wert von 7,2 titriert und mit Glutardialdehyd (9 mol / mol Hb) versetzt, nachfolgende Reaktionszeit etwa 1,5h. Nach Titration mit Natronlauge (NaOH) auf einen pH-Wert von 7,8 folgte eine Reduktion der gebildeten Schiffschen Basen mit Natriumborhydrid (10 mol NaBH₄ / mol Hb), nachfolgende Reaktionszeit etwa 0,75 h. Nach einer weiteren Titration der Lösung mit NaOH auf einen pH-Wert von 8,5 wurde das gelöste Hämoglobin mit einem 8-fachen molaren Überschuss von Methoxy-Succinimidylpropionat-Polyethylenglykol des Molekulargewichts 2000 g/mol umgesetzt, nachfolgende Reaktionszeit etwa 1 h. Mit reinem Sauerstoff erfolgte dann eine Ligandierung des vernetzten Hämoglobins. Das Milieu des gelösten Produktes wurde anschließend mit Hilfe einer Volumenausschluss-Chromatographie (Sephadex G-25 - Gel, Pharmacia. D) gegen eine wässrige ElektrolytLösung der Zusammensetzung 125 mM NaCl, 4,5 mM KCl und 20 mM NaHCO₃ gewechselt.

Messungen unter physiologischen Bedingungen (37 °C Temperatur, 40 Torr Kohlendioxid-Partialdruck und ein pH-Wert von 7,4) ergaben für das Produkt einen n50-Wert (Kooperativität) von 1,35 bei einem p50-Wert (mittlere Affinität) von 27 Torr.

### Anwendungsbeispiel 2

### Erfindungsgemäße Herstellung eines vernetzten Schweinehämoglobins mit Zusatz eines nicht-reaktiven Effektors der Sauerstoffaffinität vor der Vernetzung des Hämoglobins.

Das Produkt wurde in gleicher Weise wie in Anwendungsbeispiel 1 hergestellt, mit der einzigen Ausnahme, dass vor der Vernetzung mit Glutardialdehyd ein Zusatz von 3 mol 2,3-Bisphosphoglycerat pro Mol des zu vernetzenden, monomeren Hämoglobins erfolgt. Hierbei ergab sich unter den genannten physiologischen Bedingungen der Charakterisierung eine deutliche Verminderung des Verlustes der Kooperativität der Sauerstoffbindungsstellen des Hämoglobins, mit einem n50-Wert (als Maß für die Kooperativität) von 1,7, bei einer erhöhten mittleren Sauerstoffaffinität, ausgedrückt als ein p50 von 18 Torr.

### Anwendungsbeispiel 3 (Vergleich für 4°C)

### Erfindungsgemäße Herstellung eines vernetzten Schweinehämoglobins ohne den erfindungsgemäßen Zusatz nicht-reaktiver Effektoren bei 4°C

Das Produkt wurde in gleicher Weise wie in Anwendungsbeispiel 1 hergestellt mit dem Unterschied, dass die Herstellung bei 4°C erfolgte und die Reaktionszeiten 10x verlängert waren.

Es ergab sich unter den vorgenannten Bedingungen ein p50-Wert von 38 Torr und ein n50-Wert von 1,1.

### Anwendungsbeispiel 4

### Erfindungsgemäße Herstellung eines vernetzten Schweinehämoglobins mit Zusatz eines nicht-reaktiven Effektors der Sauerstoffaffinität vor der Vernetzung.

Ein polymeres Hämoglobin wurde ganz analog wie in Anwendungsbeispiel 3 beschrieben hergestellt, mit der Änderung, dass 2 Mol 2,3-Bisphosphoglyzerat je Mol Hämoglobin vor der Vernetzung des Hämoglobins zugesetzt wurden.
Der n50-Wert betrug 1,7, bei einem p50-Wert von 19 Torr.

### Anwendungsbeispiel 5

### Herstellung eines vernetzten Schweinehämoglobins mit Zusatz eines erfindungsgemäßen nicht-reaktiven Effektors vor der Vernetzung

Das Produkt wurde in gleicher Weise hergestellt wie in Anwendungsbeispiel 3, außer dass vor der Vernetzung 2 Mol Inositolhexaphosphat je Mol Hämoglobin zugesetzt worden waren.

Unter den oben genannten Bedingungen ergab die Analyse einen p50-Wert von 17,5 Torr und einen n50-Wert von 1,2.

### Anwendungsbeispiel 6

### Herstellung eines vernetzten Schweinehämoglobins mit Zusatz eines erfindungsgemäßen nicht-reaktiven Effektors vor der Vernetzung

Das Produkt wurde in gleicher Weise hergestellt wie in Anwendungsbeispiel 3 mit der Änderung, dass vor der Vernetzung 2 Mol Mellitsäure je Mol Hämoglobin zugesetzt waren.

Die Analyse unter den oben genannten Bedingungen ergab einen p50-Wert von 14,6 Torr und n50 von 1,5.

### Anwendungsbeispiel 7 (Vergleich)

### Herstellung eines vernetzten Schweinehämoglobins ohne den erfindungsgemäßen Zusatz eines nicht-reaktiven Effektors

Das Produkt wurde in gleicher Weise wie in Anwendungsbeispiel 1 hergestellt mit dem Unterschied, dass der Vernetzer Glykolaldehyd mit 20-fachem Überschuss verwendet wurde, dass die Reaktionszeit der Vernetzung 4h und der pH-Wert 9,1 betrug.

Unter den genannten Bedingungen ergab die Analyse einen p50-Wert von 18 mmHg und einen n50-Wert von 1,2.

### Anwendungsbeispiel 8

### Herstellung eines vernetzten Schweinehämoglobins mit Zusatz eines erfindungsgemäßen nicht-reaktiven Effektors

Das Produkt wurde in gleicher Weise hergestellt wie in Anwendungsbeispiel 7, außer dass vor der Vernetzung 2 Mol 2,3- Bisphosphoglyzerat je Mol Hämoglobin dem Reaktionsgemisch zugesetzt wurde.

Die Analyse unter den genannten Bedingungen ergab eine p50-Wert von 13 mmHg und einen n50-Wert von 1,55.

### Anwendungsbeispiel 9

### Herstellung eines vernetzten Schweinehämoglobins mit Zusatz eines erfindungsgemäßen nicht-reaktiven Effektors vor der Vernetzung

Das Produkt wurde in gleicher Weise hergestellt wie in Anwendungsbeispiel 7, mit der Veränderung, dass dem Reaktionsgemisch 2 Mol Inositolhexaphosphat je Mol Hämoglobin zugesetzt wurden.

Die Analyse unter genannten Bedingungen ergab einen p50-Wert von 21,6 mmHg und einen n50-wert von 1,6.

### Anwendungsbeispiel 10

### Herstellung eines vernetzten Schweinehämoglobins mit Zusatz eines erfindungsgemäßen nicht-reaktiven Effektors

Das Produkt wurde in gleicher Weise hergestellt wie in Anwendungsbeispiel 7, mit der Veränderung, dass dem Reaktionsgemisch 2 Mol Mellitsäure je Mol Hämoglobin zugesetzt wurden.

Die Analyse unter genannten Bedingungen ergab einen p50-Wert von 16,5 mmHg und einen n50-Wert von 1,5.

### Anwendungsbeispiel 11 (Vergleich)

### Herstellung eines vernetzten Humanhämoglobins ohne Zusatz eines nicht-reaktiven Effektors

Das Produkt wurde in gleicher Weise hergestellt wie in Beispiel 3 mit dem Unterschied einer Verwendung von Human-Hämoglobin.

Unter den genannten Bedingungen ergab die Analyse einen p50-Wert von 20 mmHg und einen n50-Wert von 1,2.

### Anwendungsbeispiel 12

### Herstellung eines vernetzten Humanhämoglobins mit Zusatz eines erfindungsgemäßen nicht-reaktiven Effektors

Das Produkt wurde in gleicher Weise hergestellt wie in Beispiel 11, außer dass vor der Vernetzung 2 Mol Bisphosphoglyzerat je Mol Hämoglobin zugesetzt waren.

Unter den oben genannten Bedingungen ergab die Analyse einen p50-Wert von 13 mmHg und einen n50-Wert von 1,4.

### Anwendungsbeispiel 13

### Herstellung eines vernetzten Humanhämoglobins mit Zusatz eines erfindungsgemäßen nicht-reaktiven Effektors

Das Produkt wurde in gleicher Weise hergestellt wie in Beispiel 11, mit der Änderung, dass vor der Vernetzung 2 Mol Inositolhexaphosphat je Mol Hämoglobin zugesetzt waren.

Unter den oben genannten Bedingungen ergab die Analyse einen p50-Wert von 12 mmHg und einen n50-Wert von 1,8.

## Patentansprüche

1. Verfahren zur Herstellung künstlicher Sauerstoffträger aus mittels eines bifunktionellen Vernetzers für Proteine, ausgewählt aus Butadiendiepoxid, Divinylsulfon, einem Diisocyanat, insbesondere Hexamethylendiisocyanat, Zyklohexyldiisocyanat und 2,5-Bisisocyanatobenzolsulfonsäure, einem Di-N-Hydroxysuccinimidylester, einem Diimidoester, oder einem Dialdehyd, insbesondere Glyoxal, dem analog reagierenden Glykolaldehyd, oder Glutardialdehyd chemisch vernetztem Hämoglobin mit verbesserten funktionellen Eigenschaften, **dadurch gekennzeichnet, dass** vor der kovalenten Vernetzung des Hämoglobins chemisch nicht reagierende Effektoren der Sauerstoffaffinität des Hämoglobins ausgewählt aus 2,3-Bisphosphoglycerat, inositolhexaphosphat, Inositolhexasulfat oder Mellitsäure zu dessen Reaktionslösung hinzu gegeben werden.

2. Verfahren nach Anspruch 1, **dadurch gekennzeichnet, dass** das Hämoglobin vom Schwein stammt.

3. Verfahren nach Anspruch 2, **dadurch gekennzeichnet, dass** als nicht-reaktiver Effektor 2,3-Bisphosphoglycerat eingesetzt wird.

4. Verfahren nach einem der Ansprüche 1 bis 4, **dadurch gekennzeichnet, dass** der nichi-reaktive Effektor der Sauerstoffaffinität des Hämoglobins vor dessen Vernetzung in einer Menge von 1 bis 20 mol pro Mol Hämoglobin zugesetzt wird.

5. Verfahren nach einem der Anspruche 1 bis 4, **dadurch gekennzeichnet, dass** das Hämoglobin vom Menschen, vom Schwein oder vom Rind stammt.

6. Verfahren nach einem der Ansprüche 1 bis 5, **dadurch gekennzeichnet, dass** das Hämoglobin in einer Konzentration von 10 bis 420 g/L eingesetzt wird.

7. Verfahren nach einem der Ansprüche 1 bis 6, **dadurch gekennzeichnet, dass** zur Vernetzung in Anwesenheit des chemisch nicht reagierenden Effektors der Sauerstoffaffinität natives Hämoglobin oder durch eine voraus gegangene chemische Modifikation derivatisiertes Hämoglobin eingesetzt wird.

8. Verfahren nach einem der Ansprüche 1 bis 7, **dadurch gekennzeichnet, dass** das Hämoglobin im desoxygenierten Zustand eingesetzt wird.

9. Verfahren nach einem der Ansprüche 1 bis 8, **dadurch gekennzeichnet, dass** das Vernetzungsreagenz in einer Menge von 3 bis 60 mol pro Mol monomeren Hämoglobins eingesetzt wird.

10. Verfahren nach Anspruch 9, **dadurch gekennzeichnet, dass** der bifunktionelle Vernetzer Glutardialdehyd ist.

11. Verfahren nach einem der Ansprüche 1 bis 10, **dadurch gekennzeichnet, dass** das während der Vernetzung mit einem chemisch nicht-reaktiven Effektor behandelte vernetzte Hämoglobin gegebenenfalls weiter chemisch modifiziert und derivatisiert wird.

12. Verfahren nach Anspruch 11, **dadurch gekennzeichnet, dass** das vernetzte Hämoglobin mit einem Polyalkylenoxid kovalent verknüpft wird.

13. Verfahren nach Anspruch 12, **dadurch gekennzeichnet, dass** das vernetzte Hämoglobin mit einem Polyethylenoxid kovalent verknüpft wird.

14. Verfahren nach einem der Ansprüche 1 bis 13, **dadurch gekennzeichnet, dass** das mit einem nicht-reaktiven Effektor behandelte, vernetzte und gegebenenfalls weiter chemisch modifizierte Hämoglobin präparativ durch Dialyse, Zentrifugation, Klär- und Ultrafiltration und/ oder präparative chromatographische Methoden gereinigt wird.

15. Verwendung eines vernetzten Hämoglobins hergestellt gemäß dem Verfahren nach einem der Ansprüche 1 bis 14 zur Herstellung eines Mittels zur intravasalen oder biomedizinischen Anwendung als künstlicher Sauerstoffträger.

16. Verwendung nach Anspruch 15, **dadurch gekennzeichnet, dass** das Mittel in Form einer pharmazeutischen Zubereitung als ein Ersatz des Blutes, oder als ein Zusatz zum Blut oder zu einer Nährlösung, im menschlichen und tierischen Organismus, in eingehen Organen, oder in biotechnischen Anwendungen verwendet wird.

17. Hämoglobin- Produkt, hergestellt gemäß einem der Ansprüche 1 bis 14.

## Claims

1. Method for the preparation of artificial oxygen carriers from haemoglobin which is crosslinked chemically by means of a bifunctional crosslinking agent for proteins chosen from butadiene diepoxide, divinyl sulfone, a diisocyanate, in particular hexamethylene-diisocyanate, cyclohexyl-diisocyanate and 2,5-bisisocyanatobenzenesulfonic acid, a di-N-hydroxysuccinimidyl ester, a diimido ester or a dialdehyde, in particular glyoxal, the analogously reacting glycol aldehyde or glutarodialdehyde, and has improved functional properties, **characterized in that** before the covalent crosslinking of the haemoglobin, chemically unreactive effectors of the oxygen affinity of the haemoglobin chosen from 2,3-bisphosphoglycerate, inositol hexaphosphate, inositol hexasulfate or mellitic acid are added to the reaction solution of the haemoglobin.

2. Method according to claim 1, **characterized in that** the haemoglobin originates from pigs.

3. Method according to claim 2, **characterized in that** 2,3-bisphosphoglycerate is used as the unreactive effector.

4. Method according to one of claims 1 to 3, **characterized in that** the unreactive effector of the oxygen affinity of the haemoglobin is added in an amount of from 1 to 20 mol per mol of haemoglobin before the latter is crosslinked.

5. Method according to one of claims 1 to 4, **characterized in that** the haemoglobin originates from man, from pigs or from cattle.

6. Method according to one of claims 1 to 5, **characterized in that** the haemoglobin is used in a concentration of from 10 to 420 g/l,

7. Method according to one of claims 1 to 6, **characterized in that**, for crosslinking in the presence of the chemically unreactive effector of the oxygen affinity, native haemoglobin or a haemoglobin derivatized by a prior chemical modification is used.

8. Method according to one of claims 1 to 7, **characterized in that** the haemoglobin is used in the deoxygenated state.

9. Method according to one of claims 1 to 8, **characterized in that** the crosslinking reagent is used in an amount of from 3 to 60 mol per mol of monomeric haemoglobin.

10. Method according to claim 9, **characterized in that** the bifunctional crosslinking agent is glutarodialdehyde.

11. Method according to one of claims 1 to 10, **characterized in that** the crosslinked haemoglobin, treated during the crosslinking with a chemically unreactive effector, optionally is modified further chemically and derivatized.

12. Method according to claim 11, **characterized in that** the crosslinked haemoglobin is linked covalently to a polyalkylene oxide.

13. Method according to claim 12, **characterized in that** the crosslinked haemoglobin is linked covalently to a polyethylene oxide.

14. Method according to one of claims 1 to 13, **characterized in that** the crosslinked haemoglobin which optionally has been modified further chemically and has been treated with an unreactive effector, is purified preparatively by dialysis, centrifugation, clarifying filtration and ultrafiltration, and/or preparative chromatographic methods.

15. Use of a crosslinked haemoglobin, prepared by the method according to one of claims 1 to 14, for the preparation of an agent for intravasal or biomedical use as artificial oxygen carrier.

16. Use according to claim 15, **characterized in that** the agent is used in the form of a pharmaceutical preparation as a replacement for blood or as an addition to the blood or to a nutrient solution, in the human and animal organism, in individual organs, or in biotechnical applications.

17. Haemoglobin product prepared according to one of claims 1 to 14.

## Revendications

1. Procédé de préparation de transporteurs d'oxygène artificiels ayant des propriétés fonctionnelles améliorées à base d'hémoglobine chimiquement réticulée à l'aide d'un agent réticulant bifonctionnel pour protéines, choisi parmi le diépoxyde de butadiène, la divinylsulfone, un diisocyanate, en particulier le diisocyanate d'hexaméthylène, le diisocyanate de cyclohexyle et l'acide 2,5-diisocyanatobenzènesulfonique, un ester de di-N-hydroxysuccinimidyle, un diimidoester, ou un dialdéhyde, en particulier le glyoxal, le glycolaldéhyde réagissant de manière analogue ou le glutaraldéhyde, **caractérisé en ce que**, avant la réticulation par covalence de l'hémoglobine, on ajoute à sa solution réactionnelle des effecteurs de l'affinité de l'hémoglobine pour l'oxygène chimiquement non réactifs, choisis parmi le 2,3-diphosphoglycérate, l'hexaphosphate d'inositol, l'hexasulfate d'inositol ou l'acide mellitique.

2. Procédé selon la revendication 1, **caractérisé en ce que** l'hémoglobine est d'origine porcine.

3. Procédé selon la revendication 2, **caractérisé en ce que** l'on utilise du 2,3-diphosphoglycérate comme effecteur non réactif.

4. Procédé selon l'une des revendications 1 à 3, **caractérisé en ce que** l'on ajoute l'effecteur non réactif de l'affinité pour l'oxygène de l'hémoglobine avant la réticulation en une quantité de 1 à 20 mol par mol d'hémoglobine.

5. Procédé selon l'une des revendications 1 à 4, **caractérisé en ce que** l'hémoglobine est d'origine humaine, porcine ou bovine.

6. Procédé selon l'une des revendications 1 à 5, **caractérisé en ce que** l'on utilise l'hémoglobine à une concentration de 10 à 420 g/l.

7. Procédé selon l'une des revendications 1 à 6, **caractérisé en ce que**, pour la réticulation en présence de l'effecteur de l'affinité pour l'oxygène chimiquement non réactif, on utilise une hémoglobine native ou une hémoglobine transformée à partir de cette dernière par une modification chimique antérieure.

8. Procédé selon l'une des revendications 1 à 7, **caractérisé en ce que** l'on utilise de l'hémoglobine à l'état désoxygéné.

9. Procédé selon l'une des revendications 1 à 8, **caractérisé en ce que** l'on utilise le réactif de réticulation en une quantité de 3 à 60 mol par mol d'hémoglobine monomère.

10. Procédé selon la revendication 9, **caractérisé en ce que** l'agent réticulant bifonctionnel est le glutaraldéhyde.

11. Procédé selon l'une des revendications 1 à 10, **caractérisé en ce que** l'hémoglobine réticulée, traitée avec un effecteur chimiquement non réactif pendant la réticulation, est éventuellement encore modifiée et transformée par voie chimique.

12. Procédé selon la revendication 11, **caractérisé en ce que** l'hémoglobine réticulée est liée par covalence à un poly(oxyde d'alkylène).

13. Procédé selon la revendication 12, **caractérisé en ce que** l'hémoglobine réticulée est liée par covalence à un poly(oxyde d'éthylène).

14. Procédé selon l'une des revendications 1 à 13, **caractérisé en ce que** l'hémoglobine traitée avec un effecteur chimiquement non réactif, réticulée et éventuellement encore modifiée par voie chimique est soumise à une purification préparative par dialyse, centrifugation, filtration de clarification et ultrafiltration, et/ou par des techniques de chromatographie préparative.

15. Utilisation d'une hémoglobine réticulée préparée par le procédé selon l'une des revendications 1 à 14 pour la préparation d'un agent destiné à l'application intravasculaire ou biomédicale en tant que transporteur d'oxygène artificiel.

16. Utilisation selon la revendication 15, **caractérisée en ce que** l'agent est utilisé sous forme d'une composition pharmaceutique comme substitut du sang, ou comme additif au sang ou à une solution nutritive, dans l'organisme humain ou animal, dans des organes isolés, ou dans des applications biotechnologiques.

17. Produit à base d'hémoglobine, préparé selon l'une des revendications 1 à 14.
